# EUROPEAN PATENT APPLICATION

(11) **EP 3 971 911 A1**
(43) Date of publication of application: **23.03.2022**
(21) Application number: 20196713.0
(22) Date of filing: 17.09.2020
(51) Int. Cl.: G16H 50/30

(54) **RISK PREDICTIONS**

(71) Applicant: Koninklijke Philips N.V., 5656 AG Eindhoven (NL)
(72) Inventor: MASCULO, Maia, 5656 AE Eindhoven (NL); VAN DEN BUIJS, Jorn, 5656 AE Eindhoven (NL); SAPORITO, Salvatore, 5656 AE Eindhoven (NL)
(74) Representative: Philips Intellectual Property & Standards

(57) **Abstract**

A risk prediction system for predicting a risk of a negative event occurring for a monitored subject. The system comprises an input interface configured to obtain case data containing a history of events for the monitored subject and a predictive model based on deep learning networks. The predictive model comprises embedding layers for embedding the case data to generate embedded data, one or more sequence learning layers configured to learn patterns from sequences of the embedded data and generate sequenced data for each layer and one or more output layers each configured to map the sequenced data of each layer to a risk prediction for a negative event occurring.

## Description

### FIELD OF THE INVENTION

The invention relates to the field of risk prediction, and more particularly to predicting a risk of a negative event occurring for a monitored subject.

### BACKGROUND OF THE INVENTION

A Personal Emergency Response System (PERS) enables an aging population to receive help quickly when an emergency situation occurs. As part of their service, PERS providers collect health-related information from their subscribers such as demographics, existing medical conditions, history of PERS utilization and more.

Some products contain predictive analytics systems which apply a combination of features and a machine learning algorithm to the data collected via PERS in order to predict the 30-day risk of emergency hospital transport. The predicted risk scores may be used to trigger preventative interventions for high risk patients. These targeted interventions are expected to both reduce costs and improve health outcomes.

However, the risk predictions may not always be accurate or reliable. Thus, there is a need to improve the reliability of the risk predictions in said products.

### SUMMARY OF THE INVENTION

The invention is defined by the claims.

According to examples in accordance with an aspect of the invention, there is provided a risk prediction system for predicting a risk of a negative event occurring for a monitored subject, the system comprising:
an input interface configured to obtain case data containing a history of events for the monitored subject; and
a predictive model based on deep learning networks comprising:
   embedding layers for embedding the case data to generate embedded data;
   one or more sequence learning layers configured to learn patterns from sequences of the embedded data and generate sequenced data for each layer; and
   one or more output layers each configured to map the sequenced data of each layer to a risk prediction for a negative event occurring.

According to some embodiments, there is proposed a deep learning-based predictive model which uses available Personal Emergency Response Systems (PERS) data to predict the associated risk(s) of negative events (i.e. unwanted/undesirable occurrences) for subject, such as a risk of falling for example. Such predictions may be used by a caregiver or care manager to undertake preventative actions for high-risk subjects for example. Also, subject-specific risk predictions may be leveraged to create subject-specific (i.e. personalized) recommendations and/or notifications.

Typical PERS have access to databases which contain case data of subjects which contains a history of events for each of the monitored subjects. Events may be falls, ambulance transport, medical treatment, administering of medication, etc. By inputting case data, including the history of events, into a deep-learning based predictive model, the model can predict the risk of negative events re-occurring.

The case data can then be input to the predictive model. The predictive model has embedding layers used to embed the case data. Each different data type from the case data may be embedded in a different embedding layer (i.e. type of negative event, situation during event, outcome of event etc.). Embedding of data is the mapping of discrete (i.e. categorical) variables to a vector of continuous numbers and can reduce the dimensionality of the data which has been embedded.

Sequence learning layers (i.e. LSTMs, GRUs etc.) then receive the outcome of the embedding layers. Sequence learning layers are used as they can process sequences of data instead of only single data points. The output of the sequence learning layers is input to output layers where the data is mapped to risk predictions. Each one of the output layers then outputs a risk prediction for a negative event occurring.

The input interface may be further configured to transform the case data to a format suitable for deep leaning networks.

In some cases, the data in the database may not have been stored in a format suitable for deep learning networks. In these cases, the data is transformed to a format suitable for deep learning networks (i.e. the data is one-hot encoded, the data is mapped to real-valued vector spaces, etc.) by the input interface before it is input to the predictive model.

The case data may contains unstructured text data and the input interface may be configured to transform the unstructured text data based on:
converting all characters in the unstructured text data to lower case; and
splitting the converted text data into tokens, wherein the embedding layers of the predictive model are configured to generate embedded text data based on the tokens,
wherein the predictive model further comprises one or more text sequence learning layers configured to learn patterns from sequences of embedded text data and generate fixed length text data, and
wherein the sequence learning layers are further configured to learn patterns from sequences of the fixed length text data and generate sequenced data further based on the fixed length text data.

PERS systems also typically allow caregivers for a monitored subject and/or a call center agent to write notes and comments for the negative events which occurred. These notes contain useful information but are generally unstructured. Text data can contain misspellings or the use of synonyms which can make it difficult for a deep learning algorithm to learn and use the text data robustly. Thus, by splitting the converted text data into tokens (i.e. sequence of n characters), the predictive model can use the text data reliably without the need of structured text data, as this may not always be possible for untrained caregivers with a PERS app.

Once the text data is split into tokens, the embedding layers of the predictive model can embed the tokens to create embedded text data. The predictive model would then have additional text sequence learning layers configured to turn sequences of the embedded text data into fixed length text data (e.g. a fixed length numerical vector). Thus, the sequence learning layers can reliably learn patterns from the fixed length text data without being largely dependent on the spelling of words, the use of the same words (e.g. no synonyms), the use of abbreviations etc.

The sequence learning layers may be bidirectional long short term memory layers.

Bidirectional long short term memories can get information from past and future states simultaneously, thus increasing the information available to the deep learning network.

The system may further comprise an alert generation module configured to generate alerts to a caregiver for the monitored subject based on one or more of:
a risk prediction being greater than a high risk threshold value;
the sum of the risk predictions being greater than a sum threshold value;
an increase in the risk prediction of an outcome; and
an increase in the sum of the risk predictions.

For example, if the risk prediction for a fall is above 50%, the caregiver for the monitored subject may be alerted to be vigilant of the monitored subject. Alternatively, the risk prediction for a fall may only be 30%, but the risk for all other negative events may also be 30%, thus the overall probability of any negative event occurring could be much higher than 30% (the real probability of any event occurring will depend on the interdependencies between each event). In this case, the caregiver may be alerted that any negative event may occur and which of these events is the most likely. Similarly, an increase in risk compared to a previous risk prediction can also be alerted to a caregiver (i.e. send alert IF risk increase is greater than 10% in a week). An alert can also, or alternatively, be sent to a PERS call center agent.

The system may further comprise a content recommendation module, wherein the content recommendation module is configured to select relevant content from a content library for the monitored subject based on:
identifying high risk negative events for the monitored subject based on the corresponding risk predictions having a value greater than a high risk threshold value;
selecting relevant content from the content library based on the high risk negative events for the monitored subject, wherein content in the content library contains negative event tags corresponding to the topics of the content and wherein selecting relevant content for the monitored subject is based on selecting content from the library with negative event tags corresponding to the high risk negative events of the monitored subject.

For example, if the monitored subject has a high risk of falling (i.e. higher than 50%), the content recommendation module could choose an article related to fall prevention strategies. It may also choose advertisements which are related to fall prevention, such as a vitamin D supplement advertisement.

The content (i.e. articles, advertisements etc.) in the library are given tags which relate to possible negative events. For example, an article about fall prevention would have a "fall" tag. Relevant content for a subject can then be chosen based on looking for content with tags which relate to the negative events with the highest predicted risk for the monitored subject.

A further input to the predictive model may be time elapsed since each event in the history of events for the monitored subject.

Sequence learning layers typically assume events in a sequence are symmetrically distributed in time. By including the time elapsed since each negative event as an input to the predictive model, the sequence learning layers consider the time differences between events and the patterns (or lack thereof) in time distribution of said events.

The invention also provides a method for predicting a risk of negative events occurring for a monitored subject, the method comprising:
obtaining case data containing a history of events for the monitored subject;
inputting the case data into embedding layers for embedding the case data to generate embedded data;
inputting the embedded data into one or more sequence learning layers, wherein the sequence learning layers are configured to learn patterns from sequences of the embedded data and generate sequenced data for each layer; and
inputting the sequenced data to one or more output layers, wherein the output layers are configured to map the sequenced data to a risk prediction and wherein each output layer outputs a risk prediction for a negative event occurring.

The method may further comprise transforming the case data to a format suitable for deep leaning networks.

The case data may contain unstructured text data and transforming the unstructured text data may comprise:
converting all characters in the unstructured text data to lower case; and
splitting the converted text data into tokens, wherein the embedding layers are configured to generate embedded text data based on the tokens,
wherein the method further comprises inputting the embedded text data to one or more text sequence learning layers configured to learn patterns from sequences of embedded text data and generate fixed length text data, and
wherein the sequence learning layers are further configured to learn patterns from sequences of the fixed length text data and generate sequenced data further based on the fixed length text data.

The sequence learning layers may be bidirectional long short term memory layers.

The method may further comprise generating alerts to a caregiver for the monitored subject based on one or more of:
a risk prediction being greater than a high risk threshold value;
the sum of the risk predictions being greater than a sum threshold value;
an increase in the risk prediction of a negative event; and
an increase in the sum of the risk predictions.

The method may also further comprise:
identifying high risk negative events for the monitored subject based on the corresponding risk predictions having a value greater than a high risk threshold value; and
selecting relevant content from a content library based on the high risk negative events for the monitored subject, wherein content in the content library contains negative event tag corresponding to the topic of the content and wherein selecting relevant content for the monitored subject is based on selecting content from the library with negative event tags corresponding to the high risk negative events of the monitored subject.

The method may also further comprise inputting the time elapsed since each event in the history of events into the sequence learning layers.

The invention also provides a computer program product comprising computer program code means which, when executed on a computing device having a processing system, cause the processing system to perform all of the steps of the methods as described above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a schematic representation of a risk prediction system according to a proposed embodiment;
Fig. 2 shows an example of a data table for the case data of Fig. 1;
Fig. 3 shows a schematic representation of the risk prediction system of Fig. 1, wherein the case data contains unstructured data;
Fig. 4 shows a schematic representation of an example architecture of the predictive model of Fig. 1 used to predict a subject's risk of falling and of emergency transport; and
Fig. 5 shows a schematic representation of a risk prediction system according to another embodiment.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a risk prediction system for predicting a risk of a negative event occurring for a monitored subject. The system comprises an input interface configured to obtain case data containing a history of events for the monitored subject and a predictive model based on deep learning networks. The predictive model comprises embedding layers for embedding the case data to generate embedded data, one or more sequence learning layers configured to learn patterns from sequences of the embedded data and generate sequenced data for each layer and one or more output layers each configured to map the sequenced data of each layer to a risk prediction for a negative event occurring.

A history of events could contain negative events such as falls, seizures, breathing problems etc. as well as other events which affect the health of the monitored subject, such as medical visits, changes in prescription medicines, physical activity, eye tests, etc.

The risk prediction system can be scaled to output any number of negative outcome predictions. The same predictive model can be extended to predict risk scores for any number of desired outcomes such as respiratory problems, chest pain, seizures, etc. These risk scores are complementary to the overall emergency hospital transport risk as they provide additional information that may be used by the clinician to choose the appropriate intervention.

The predictive system has been validated by creating and evaluating a single model which accurately predicts the subject's risk of falling and of emergency transport, achieving superior performance than existing methods for both outcomes.

The case data may have to be transformed to a format suitable for deep leaning networks in some cases. For example, for numerical values, the scale of the values will affect the training of the prediction model and thus typically numerical values are scaled or normalized. Unscaled input variables can result in a slow or unstable learning process and unscaled target variables (i.e. target outputs during the training of the model) can result in instabilities causing the learning process to fail. The scaling process will depend on the particular architecture and functions used in the predictive model.

Additionally, for categorical values such as True or False values, the vales may need to be, for example, one hot encoded if they are not numerical and/or they are text-based variables. Deep learning algorithms use numerical weightings in their functions and, as a result, using text-based data is typically more difficult to learn than numerical data. For example, True or False data could be transformed to True = 1 and False = 0 or True = [10] and False = [01].

The input interface could be configured to perform the transformation. For example, the input interface could perform one hot encoding, input data normalization and/or any other known transformations typically used in for formatting data for deep neural networks.

Additionally, the case data could contain unstructured text data and the input interface would then be configured to transform the unstructured text data to a suitable format. Unstructured text data typically requires a lot of training and is very sensitive to different writing styles, use of synonyms, spelling mistakes etc.

Structured data typically comprises pre-determined values (e.g. Headache = yes / no / don't know) or ranges (e.g. age = 0 - 120) which can be selected. Thus, the predictive model can be easily trained on these known pre-determined answers. However, for unstructured data, anything could be input. A simple example of this is asking the question: "Do you have a headache?" There are many ways to answer this question in the affirmative, such as: "Yes, I do", "Yeah", etc., as well as the possibility of spelling errors, different use of capital letters, slang terms, etc. Thus, it can be very difficult for a deep learning algorithm to learn all of these variations. Unstructured text data is thus an input of text data in which a user can freely choose what to type and is not obliged to choose from pre-determined values (i.e. an empty text box).

The input interface can convert all characters in the unstructured text data to lower case and split the converted text data into tokens. Tokens are n-sized vectors containing n characters from the unstructured text data. For example, for n = 3, the word "fallen" would turn into two tokens: [fal] [len]. It is much easier for a deep neural network to learn patterns within vectors of fixed length and consistent formatting (i.e. no upper case letters) compared to learning patterns of, for example, words in a sentence.

The embedding layers of the predictive model can then generate embedded text data based on the tokens. The predictive model could also have one or more text sequence learning layers configured to learn patterns from sequences of embedded text data and generate fixed length text data. The fixed length text data would then be input to the sequence learning layers such that they can learn patterns from sequences of the fixed length text data and the embedded data. The sequence learning layers would then generate sequenced data based on the fixed length text data and the embedded data.

In essence, the predictive system can extract information from unstructured data modalities, such as free text. A proposed predictive system is designed to extract information from a caregiver database (e.g. text notes written by the call center agent to document each case, notes written by a family member etc.) in order to make risk predictions. These case notes contain richer information about a subject's history than is possible to represent with structured variables. For example, they may contain contextual information and details about past incidents, symptoms, information about the subject's care circle, etc. Therefore, the ability to extract relevant information from this unstructured data source improves the predictive power of the predictive model.

Additionally, the predictive system can be integrated with a caregiver app. Since the predictive model could extract information from various data types, including free text, the data provided by caregivers via an app can be used as input to improve the predictive power of the model.

The sequence learning layers and/or the text sequence learning layers could be bidirectional long short term memory (BLSTM) layers. The principle of BLSTMs is to split the neurons of a regular LSTM into two directions, one for positive time direction (forward states), and another for negative time direction (backward states). Those two states' output are not connected to inputs of the opposite direction states.

BLSTMs are especially useful when the context of the input is needed. In the current application it is useful for the BLSTM to know the tokens which appear before and after any other token. In practice, the inputs for the BLSTMs will be the embedded vectors and not the token vectors. However, the function of BLSTMs is much better understood with practical examples. Thus, the next example will be explained with token vectors.

It is useful for the BLSTM to know that the token [1-do] (token of size n=4) is preceded by the token [-fal] and is succeeded by the token [wn--] (for the purpose of the example, the dash [-] is used to represent a space or any punctuation). In this example, the combination of [-fal][1-do][wn--] has more meaning than the single token [1-do].

Sequence learning layers (such as BLSTMs) typically assume no time dependence (or constant time change) between inputs in a sequence. For a non-time dependent sequence (e.g. the tokens) this does not matter. However, for time dependent sequences (e.g. events occurred to the subject), this assumption is not true.

For example, if the subject typically falls a second time soon after falling a first time, the time difference between these falls is an important aspect of predicting how likely it is for the subject to fall again. Thus, the time elapsed since each event in the history of events could also be input to the predictive model.

The predictive system could also have an alert generation module which generates alerts to a caregiver for the monitored subject. The caregiver could, for example, be a family member, a close friend or a call center operator. An alert would be generated if a risk prediction is greater than a high risk threshold value, if the sum of the risk predictions is greater than a sum threshold value, if there is an increase in the risk prediction of an outcome and/or if there is an increase in the sum of the risk predictions.

For example, if there is a high risk that the subject will fall down within the next few weeks it is important to alert a family member or someone who may live with or near the subject in order to be attentive to possible falls. Alternatively, if the subject is likely to incur respiratory problems in the near future the alert generation module could alert the subject's physician to provide preventive measures before the problems occur.

In some cases, the subject could also be alerted of a high risk of a possible negative events.

The predictive model could also have a content recommendation module. The content recommendation module is configured to select relevant content from a content library for the monitored subject. A high risk negative event is identified by comparing the risk prediction of the negative event to a high risk threshold value. If the risk prediction is higher than the high risk threshold value, relevant content is selected from the content library based on the high risk negative events for the monitored subject.

Content in the content library contains negative event tags corresponding to the topics of the content. Thus, selecting relevant content for the subject is based on selecting content from the library with negative event tags corresponding to the high risk negative events of the monitored subject.

The content library can have articles on improving and/or managing conditions, advertisements for products which can reduce the risk of negative events etc. As such, tailored content can be provided to the subject (i.e. in a health app) thus improving the user experience and engagement with the app.

Alternatively, the predictive system could have an advertisement module separate from the content recommendation module, which would be configured to choose and present advertisements for the subject based on the same concept as the content recommendation module.

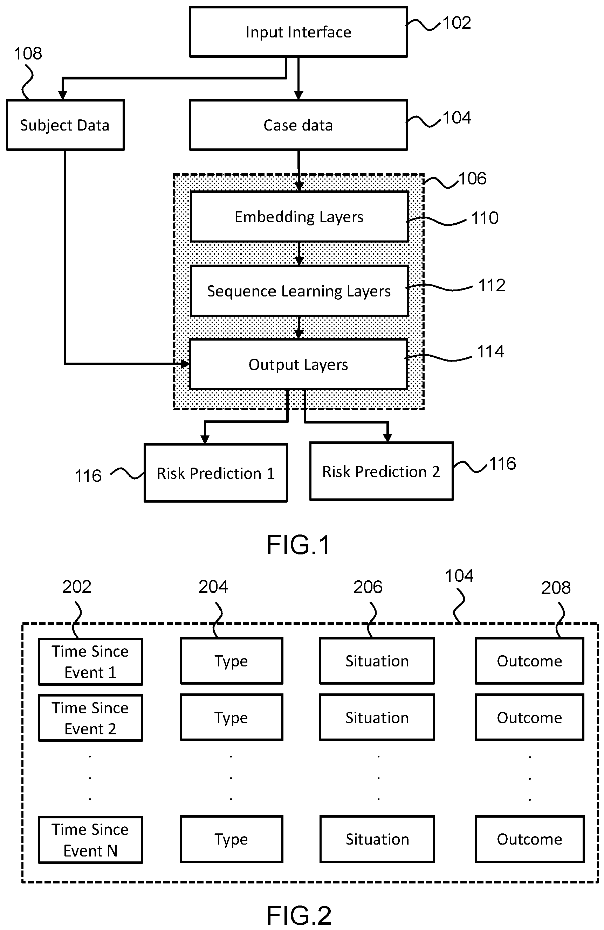

Fig. 1 shows a schematic representation of a risk prediction system according to an exemplary embodiment. The input interface 102 retrieves data of a specified subject from databases (i.e. caregiver databases, event databases etc.) and applies different preprocessing operations to the data depending on their type. If available, data from the caregiver app database is retrieved and linked to the event data using subject ID information. In this example, subject data 108 (i.e. age, height, weight, medical conditions etc.) and case data 104 containing historic event data is retrieved for a subject from said databases.

The main operations performed on different data types are:
(i) Medical conditions - A list of self-reported medical conditions is converted into a fixed-length binary vector where each element of the vector indicates whether or not a medical condition was reported by the subscriber.
(ii) Categorical variables - All categorical variables (i.e. subscriber's region, subscription type and enrolment program) are encoded using integer values. In the prediction model 106 these integer values are mapped to a real-valued vector space using embedding layers 110. Alternatively, the categorical variables are one-hot-encoded.
(iii) History of events - The subject's history of events is a sequence of timestamped events where each event is documented using categorical variables (e.g. type, situation and outcome of the event) and possibly a text note. The historic events are sorted in chronological order based on their timestamp. The categorical variables of each event are encoded using the process described above for categorical variables.

The case data 104 (including a history of events) is input into the predictive model 106. The predictive model 106 has embedding layers 110, sequence learning layers 112 and output layers 114. The embedding layers 110 are for embedding the case data 104. Often when using data pre-processed for deep learning networks (e.g. after being one-hot encoded), each data point can have a large dimensionality. Thus, the embedding layers 110 can replace the high dimensionality data points with lower dimensionality ones.

The embedded data is then input into sequence learning layers 112 in the predictive model 106. The sequence learning layers 112 can learn patters of sequences of the embedded data points. In an oversimplified example, if the sequence of data points provided to the sequence learning layers 112 is [0][0][1][1][0][0][0][0][1][0][1][0][0], where a [1] signifies a fall on a specified day and a [0] signifies no fall on the specified day, the sequence learning layers 112 will begin to recognize that a second fall is likely to occur one or two days after a first fall.

The sequence learning layers 112 can also learn patterns between two different events (e.g. typically no falls on the days after a physical therapy session). The historic event in the case data 104 can include a history of negative events and of events which may affect the health of the subject (i.e. doctor appointments, change of prescription, therapy sessions etc.).

The output of the sequence learning layers 112 is then input into output layers 114. Additionally, the subject data 108 (medical conditions, age, weight, height etc.) can also be input to the output layers 114 of the predictive model 106. The output layers 114 map the output of the sequence learning layers 112 to predictions 116 on risk scores for various negative events occurring (in this case, two negative events). The mapping may be dependent on the subject data 108 (e.g. if the subject has a twisted ankle the risk of falling may be generally higher or, the older the subject is, the higher the risk of certain negative events).

Fig. 2 shows an example of a data table for the case data 104 of Fig. 1. Each row in the table has data for a single event. The first column is the time since each event occurred 202 (alternatively this could be a time stamp for each event). The following three columns show categorical data giving the type of event which occurred 204, the situation in which the subject was in when the event occurred 206 and the outcome of the event 208. An example of a row could be: [24 hours] [Respiratory problems] [Home] [Hospitalization]. Other examples of categorical data that could be associated with events include reported signs and symptoms or diagnosed conditions (e.g. using a coding scheme such as ICD-10). In general, different PERS will use different variables to document each case.

The case data 104 could also have a column for case notes. These notes typically have unstructured text from a clinician, call operator and/or caregiver for each event (e.g. doctors notes after a check up).

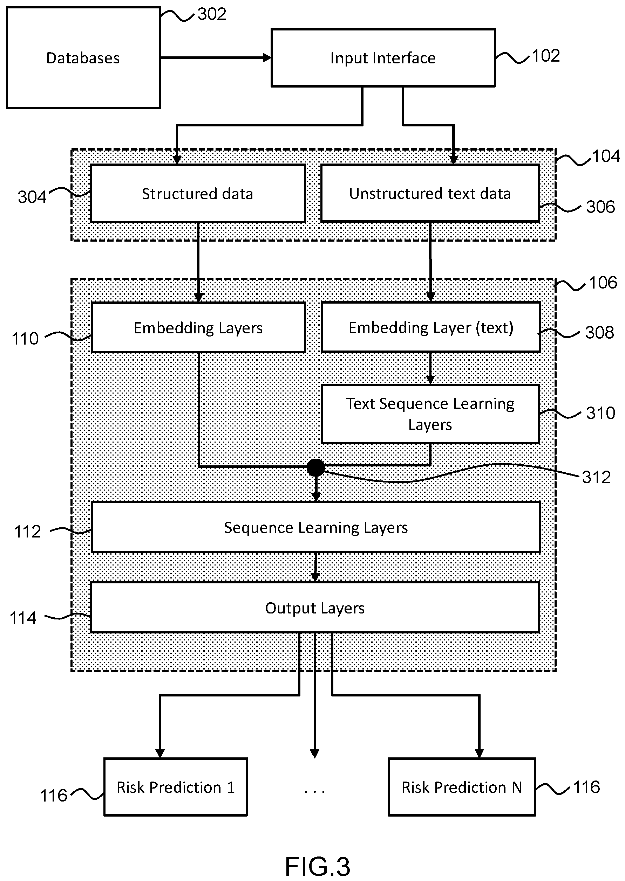

Fig. 3 shows a schematic representation of the risk prediction system when the case data 104 contains unstructured data 306. The databases 302 containing the case data 104 could also contain case notes for each event. When the input interface obtains unstructured text data 306 (i.e. case notes), they must be pre-processed appropriately before being input to the predictive model 106. The case notes 306 associated with each case are lowercased and split into individual tokens.

As with the categorical variables, each unique token is encoded using integer values which are then mapped by the predictive model 106 to a real-valued vector space using one or more text embedding layers 308. As a pre-training step, text embedding layers 308 may be created for each token in an unsupervised manner.

The embedded tokens are then input into text sequence learning layers 310. The output of the text sequence learning layers 310 is a numerical vector representation of each case note which can be combined (e.g. by concatenating) at point 312 with the structured data 304 (i.e. categorical values), after it has been embedded with the embedding layers 110, to the same case.

The concatenated data is then input into sequence learning layers 112. Output layers 114 are then used to map the output of the sequence learning layers 112 to predicted risk values 116 for a number N of negative events. The output of the sequence learning layers 112 can also be combined with the subject data 108 (e.g. age, weight, height etc.) before it is input into the output layers 114 (not shown in Fig. 3).

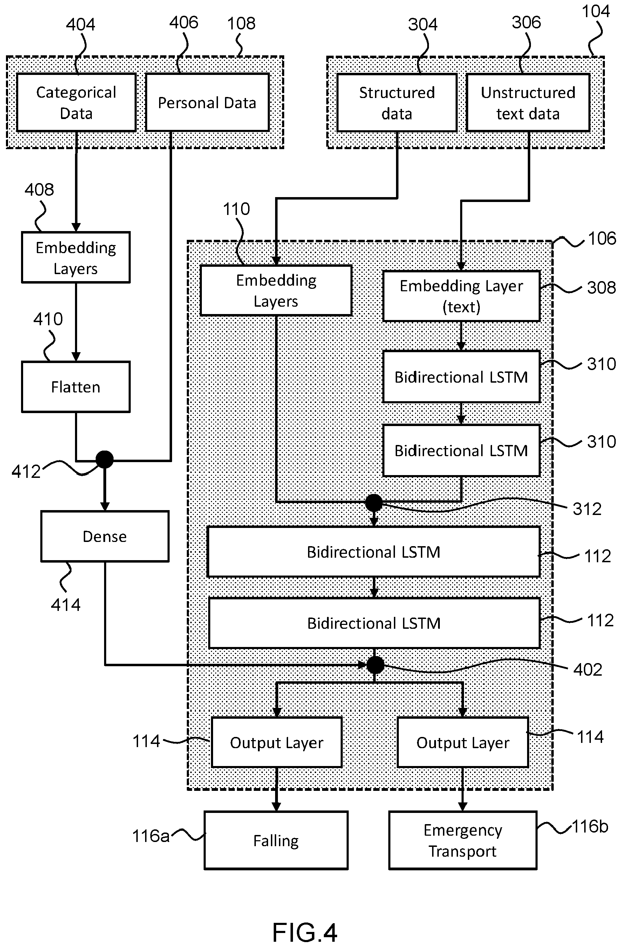

Fig. 4 shows a schematic representation of an example architecture of the predictive model 106 used to predict a subject's risk of falling and of emergency transport.

The structured data 304 (i.e. categorical data) and the unstructured text data 306 (i.e. case note tokens) from the case data 104 are input into different embedding layers. The text embedding layer 308 (for the unstructured data 306) is configured to perform word embedding on the tokens of unstructured text data. Sequences of the embedded text data (e.g. a whole sentence divided into tokens or the whole case note divided into tokens) is then input into two consecutive time distributed bidirectional LSTMs 310 in order to generate a fixed-length vector representation of each sequence.

A numerical representation of each event is obtained by concatenating the structured data 304 (after it has been input into the embedding layers 110) of all associated categorical variables (e.g. type, situation and outcome) and fixed-length vector representation of the unstructured text data at point 312. Additional bidirectional LSTM layers 112 are then used to process the sequence of events. Since LSTMs are designed with the assumption that the elements of the sequence are uniformly spaced, the time elapsed since each event is provided as an additional input to the predictive model (not shown in Fig. 4).

The output of the bidirectional LSTM layers 112 is then combined (i.e. concatenated) at point 402 with the subject data 108. However, the subject data 108 may first need to be pre-processed.

Subject data 108 can contain personal data 406 and categorical values 404. The personal data 406 contains real valued and binary variables such as the patient's age, gender, self-reported medical conditions and time on PERS service. The categorical data 404 (such as the user's home location) is first embedded by embedding layers 408 and is then flattened by a flatten layer 410 (which reshapes the data so it can be concatenated). The flattened data iis then concatenated at point 412 with the personal data 406. The concatenated categorical data and personal data is then processed by stacked dense layers 414.

The output of the bidirectional LSTM layers 112 and dense layers 414 are combined at point 402 and used as input the output layers 114. In this example there are two output layers 114, one for mapping the data to a predicted risk score of the subject falling 116a and the other to a predicted risk score for the subject needing emergency transport 116b.

Before the system can be deployed, the predictive model 106 is trained based on historical data. The prediction task is formulated as a set of binary classification problems which can be stated as follows: given the data available (history of events and/or subject data) in a certain observation time window, predict the risk of a set of outcomes occurring in a given prediction time window.

The training dataset may be constructed using a walk forward approach. A first prediction time window and a first observation time window are defined from which the labels (outcome variables) and the input data for prediction are extracted respectively. Then, both the prediction and observation windows are shifted in time by the same amount and new labels and input data are extracted. This process is repeated until the entire historical data is used. Finally, all label-input pairs are combined in a single dataset that can be used to develop and test the predictive model. In experiments, 30-day prediction windows and 2-year observation windows were used.

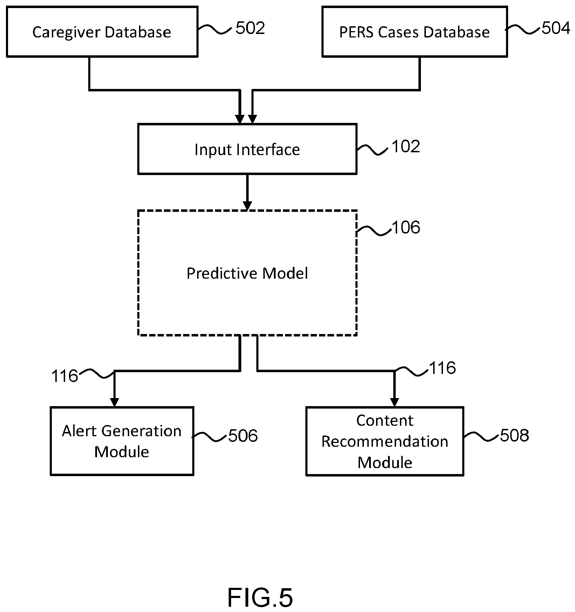

Fig. 5 shows a schematic representation of a risk prediction system with an alert generation module 506 and a content recommendation module 508.

The risk predictions 116 from the predictive model 106 are input into the alert generation module 506. A threshold is defined for each negative event and an alert is generated for a subject if any of their predicted risk scores 116 is above its corresponding threshold.

Alternatively, an alert is generated if the sum or the average of the risk scores 116 for a subscriber is above a specified threshold.

Alerts may be generated based on the detection of an increase in risk score 116. This may be done by applying a threshold on the difference between the current predicted risk scores 116 and the risk scores 116 predicted in a previous time; or by applying trend detection techniques to the sequence of predicted risk scores 116.

The risk predictions 116 from the predictive model 106 are also input into a content recommendation module 508. The content recommendation module 508 uses the predicted risk scores 116 in order to select relevant content and advertisements for the subject and/or the caregivers (e.g. content shown on a caregiver app).

Alternatively, the risk prediction system may have a separate content recommendation module 508 and an advertisement module. For example, if a subject is determined to have a high fall risk, the content recommendation module 508 may select an article about falls prevention strategies to be displayed on an app, while the advertisement module may select a daily vitamin D supplement (which is a potential fall prevention intervention) advertisement.

The content recommendation module 508 and/or the advertisement module may be rule-based. Each article and advertisement may be tagged with tags related to the negative events predicted by the predictive model 106, e.g. a falls prevention article would be tagged with the "fall" tag. Whenever a subscriber is flagged as high fall risk, the modules would recommend a random (unread) article which has the "fall" tag.

The content recommendation module 508 and advertisement module may be based on machine learning algorithms trained on historical app data.

In summary, an example of the invention describes a system comprised of:
(i) A database storing information about subscribers to a PERS service, potentially including data of several types such as demographics, medical conditions, location, history of PERS service utilization, etc.
(ii) A database storing usage information of a caregiver app. This usage information may include, for example, text notes entered by the caregivers of a certain PERS subscriber.
(iii) An input interface that retrieves the PERS and app data related to specified subscribers from the databases and pre-processes each data element according to its type. The input interface outputs the data from each specified subscriber in a standard format which is suitable for further processing.
(iv) A deep learning-based predictive model, including a method to train such model, which takes the pre-processed data from the specified subscribers as input and outputs risk scores for a number of outcomes such as falls, respiratory problems, emergency hospital transport, etc.
(v) An alert generation module which generates alerts for a particular subscriber based on the various risk scores provided by the predictive model for the subscriber and a means to present the risk scores and alerts to a care manager, e.g. via a dashboard, such that appropriate interventions may be triggered for the subscribers flagged as high risk.
(vi) Content recommendation and advertising modules which use the predicted risk scores in order to select relevant content and advertisements for the users of the caregiver app.

A machine-learning algorithm is any self-training algorithm that processes input data in order to produce or predict output data. Here, the input data comprises subject data and a history of events for a subject and the output data comprises risk predictions for negative events.

Suitable machine-learning algorithms for being employed in the present invention will be apparent to the skilled person. Examples of suitable machine-learning algorithms include decision tree algorithms and artificial neural networks. Other machine-learning algorithms such as logistic regression, support vector machines or Naive Bayesian models are suitable alternatives.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. ±1%) to the training output data entries. This is commonly known as a supervised learning technique.

For example, where the machine-learning algorithm is formed from a neural network, (weightings of) the mathematical operation of each neuron may be modified until the error converges. Known methods of modifying a neural network include gradient descent, backpropagation algorithms and so on.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A risk prediction system for predicting a risk of a negative event occurring for a monitored subject, the system comprising:
an input interface (102) configured to obtain case data (104) containing a history of events for the monitored subject; and
a predictive model (106) based on deep learning networks comprising:
embedding layers (110) for embedding the case data (104) to generate embedded data;
one or more sequence learning layers (112) configured to learn patterns from sequences of the embedded data and generate sequenced data; and
one or more output layers (114) each configured to map the sequenced data of each layer to a risk prediction (116) for a negative event occurring.

2. The system of claim 1, wherein the input interface (102) is further configured to transform the case data (104) to a format suitable for the deep learning networks.

3. The system of claim 2, wherein the case data (104) contains unstructured text data (306) and the input interface (102) is configured to transform the unstructured text data (306) based on:
converting all characters in the unstructured text data (306) to lower case; and
splitting the converted text data into tokens,
wherein the predictive model (106) further comprises text embedding layers (308) configured to generate embedded text data from the tokens,
wherein the predictive model (106) further comprises one or more text sequence learning layers (310) configured to learn patterns from sequences of embedded text data and generate fixed length text data, and
wherein the sequence learning layers (112) are further configured to generate sequenced data further based on the fixed length text data.

4. The system of any one of claims 1 to 3, wherein the sequence learning layers (112) comprise bidirectional long short term memory layers.

5. The system of any one of claims 1 to 4, further comprising an alert generation module (506) configured to generate one or more alerts for a caregiver for the monitored subject based on one or more of:
a risk prediction (116) being greater than a high risk threshold value;
the sum of the risk predictions (116) being greater than a sum threshold value;
an increase in a risk prediction (116) for a negative event; and
an increase in the sum of the risk predictions (116).

6. The system of any one of claims 1 to 5, further comprising a content recommendation module (508) configured to select relevant content from a content library for the monitored subject based on:
identifying high risk negative events for the monitored subject based on the corresponding risk predictions (116) having a value greater than a high risk threshold value;
selecting relevant content from the content library based on the high risk negative events for the monitored subject, wherein content in the content library contains negative event tags corresponding to the topics of the content and wherein selecting relevant content for the monitored subject is based on selecting content from the library with negative event tags corresponding to the high risk negative events of the monitored subject.

7. The system of any one of claims 1 to 6, wherein a further input to the predictive model (106) comprise a time elapsed since each event (202)) in the history of events.

8. A method for predicting a risk of negative events occurring for a monitored subject, the method comprising:
obtaining case data (104) containing a history of events for the monitored subj ect;
inputting the case data (104) into embedding layers (110) for embedding the case data (104) to generate embedded data;
inputting the embedded data into one or more sequence learning layers (112), wherein the sequence learning layers (112) are configured to learn patterns from sequences of the embedded data and generate sequenced data for each layer; and
inputting the sequenced data to one or more output layers (114), wherein the output layers (114) are configured to map the sequenced data to a risk prediction (116) and wherein each output layer (114) outputs a risk prediction (116) for a negative event occurring.

9. The method of claim 8, further comprising transforming the case data (104) to a format suitable for the deep leaning networks.

10. The method of claim 9, wherein the case data (104) contains unstructured text data (306) and wherein transforming the unstructured text data (306) comprises:
converting all characters in the unstructured text data (306) to lower case; and
splitting the converted text data into tokens,
wherein tokens are input into text embedding layers (308) configured to generate embedded text data from the tokens,
wherein the embedded text data is input into text sequence learning layers (310) configured to learn patterns from sequences of embedded text data and generate fixed length text data, and
wherein the sequence learning layers (112) are further configured to generate sequenced data further based on the fixed length text data.

11. The method of any one of claims 8 to 10, wherein the sequence learning layers (112) comprise bidirectional long short term memory layers.

12. The method of any one of claims 8 to 11, further comprising generating one or more alerts for a caregiver for the monitored subject based on one or more of:
a risk prediction (116) being greater than a high risk threshold value;
the sum of the risk predictions (116) being greater than a sum threshold value;
an increase in a risk prediction (116) for a negative event; and
an increase in the sum of the risk predictions (116).

13. The method of any one of claims 8 to 12, further comprising:
identifying high risk negative events for the monitored subject based on the corresponding risk predictions (116) having a value greater than a high risk threshold value; and
selecting relevant content from a content library based on the high risk negative events for the monitored subject, wherein content in the content library contains negative event tag corresponding to the topic of the content and wherein selecting relevant content for the monitored subject is based on selecting content from the library with negative event tags corresponding to the high risk negative events of the monitored subject.

14. The method of any one of claims 8 to 13, further comprising inputting the time elapsed since each event (202) in the history of events into the sequence learning layers (112).

15. A computer program product comprising computer program code means which, when executed on a computing device having a processing system, cause the processing system to perform all of the steps of the method according to any of claims 8 to 14.
